# EUROPEAN PATENT APPLICATION

(11) **EP 2 095 820 A1**
(43) Date of publication of application: **02.09.2009**
(21) Application number: 06843266.5
(22) Date of filing: 26.12.2006
(51) Int. Cl.: A61K 35/74, A23C 9/13, A61K 31/164, A61K 35/20, A61K 38/00, A61P 17/00, C12N 1/20

(54) **FERMENTED MILK FOR IMPROVING AND/OR TREATING SKIN AND METHOD FOR PRODUCING THE SAME**

(71) Applicant: Meiji Dairies Corporation, Tokyo 136-8908 (JP)
(72) Inventor: KANO, Hiroshi, Odawara-shi Kanagawa 250-0862 (JP); NOMA, Teruyuki, Odawara-shi Kanagawa 250-0862 (JP); IKEGAMI, Shuji, Odawara-shi Kanagawa 250-0862 (JP); ITO, Hiroyuki, Odawara-shi Kanagawa 250-0862 (JP); ISAWA, Kakuhei, Odawara-shi Kanagawa 250-0862 (JP)
(74) Representative: Bentz, Jean-Paul
(86) International application number: PCT/JP2006/325882
(87) International publication number: WO 2008/078387

(57) **Abstract**

Object

The present invention provides a composition and fermented milk, which is derived from natural product, is highly safe, exhibits improvement effect and/or therapeutic effect on skin, and comprises fermented lactic acid bacteria excellent in manufacturability.

Solving Means

Disclosed are: use of *Streptococcus thermophilus* OLS3059 in skin improvement and/or treatment; a composition for skin improvement and/or treatment comprising *Streptococcus thermophilus* OLS3059 and/or a culture thereof; food and drink comprising the composition; fermented milk for skin improvement and/or treatment prepared by using *Lactobacillus delbrueckii* subspecies *bulgaricus* and *Streptococcus thermophilus* OLS3059; and the fermented milk for skin improvement and/or treatment further comprising collagen peptide and/or ceramide.

## Description

### Technical Field

The present invention relates to a composition having high improvement effect and/or therapeutic effect on skin, food and drink and fermented milk using the same and particular to a composition and fermented milk comprising lactic acid bacteria excellent in manufacturability.

### Background Art

As compositions and food and drink for the purpose of skin improvement, treatment etc., there are those containing lactic acid bacteria or cultures thereof.

Related arts directed to such compositions and food and drink are described below:
an atopic dermatitis suppressant and food using specific lactic acid bacteria (Patent Document 1);
a humectant having an improvement effect on skin, which uses a culture of specific lactic acid bacteria and a cosmetic composition effective at prevention and treatment of rough skin and dry skin and giving "fair complexion" and "supple state" to skin (Patent Document 2);
use of specific lactic acid bacteria and cultures thereof in well-balanced immune system of skin as well as food or the like comprising lactic acid bacteria and a culture thereof (Patent Document 3);
weight-reducing food expected to achieve improvement in laxation, recovery from fatigue, alleviation of stiff neck, etc. in addition to improvement in skin by using lactic acid bacteria or a culture thereof in combination collagen peptide or ceramide (Patent Document 4).

As described above, it was found that lactic acid bacteria and cultures thereof were found to have an improvement effect and therapeutic effect on skin, and actually, industrial application thereof has conventionally been expected. Some specific studies have been made, and it is estimated that there are actually some commercial products thereof.

However, examination of the relationship between lactic acid bacteria and skin or the relationship between a composition comprising, for example, collagen peptide and ceramide added to lactic acid bacteria and skin is insufficient in conventional studies. Accordingly, there is a shortage of scientific grounds for the improvement effect of lactic acid bacteria or fermented milk on skin etc.

Conventional lactic acid bacteria having an improvement effect on skin etc., when producing fermented milk, do not form curd, or the time for forming curd is significantly long. In this case, lactic acid bacteria having an improvement effect on skin but poor in manufacturability of fermentedmilk and lactic acid bacteria excellent in manufacturability for forming curd are separately managed and the fermentation time is prolonged, and thus the manufacturing time is increased as a whole. Conventional lactic acid bacteria having an improvement effect on skin etc. are not viable bacteria but killed bacteria or highly thermostable limited bacteria. That is, conventional lactic acid bacteria having an improvement effect on skin etc. are not suitable for large-scale production (commercial production) of fermented milk etc.

Roughly speaking, manufacturing processes shown below will be necessary when conventional lactic acid bacteria regarded as having an improvement effect on skin etc. are used to produce fermented milk. That is, (1) lactic acid bacteria having an improvement effect on skin etc. are cultured alone. (2) The lactic acid bacteria (cultured microbial bodies) are concentrated to a predetermined density by centrifugation or separation through a membrane, or their culture is sterilized by heating or dried in order to improve the storage stability of the lactic acid bacteria, depending on the situation. (3) Other lactic acid bacteria forming curd, their concentrated cultured microbial bodies, or those sterilized by heating or dried are added to blended milk before fermentation and subjected to mixed culture.

These manufacturingprocesses are complicated to regulate properly, and the manufacturing costs and the manufacturing time are increased, and there is a high probability of contamination. Fermented milk suiting consumer' s liking and excellent in taste is hardly obtainable.
Patent Document 1: Japanese Patent Application Laid-Open No. 2000-095697
Patent Document 2: Japanese Patent Application Laid-Open No. 2003-081808
Patent Document 3: Japanese Patent Application Laid-Open No. 2004-510740
Patent Document 4: Japanese Patent Application Laid-Open No. 2004-254632

### Disclosure of the Invention

### Problem to be Solved by the Invention

The present invention was made in view of the related art and problems described above, and the object of the present invention is to provide a composition which is derived from natural products, is highly safe and has high improvement effect and/or therapeutic effect on skin, a composition suitable for use in skin improvement and/or treatment, food and drink and fermented milk having high improvement effect and/or therapeutic effect on skin, comprising the composition, particularly a composition and fermented milk comprising lactic acid bacteria excellent in manufacturability.

### Means for Solving the Problem

As a result of extensive study in view of the problem described above, the present inventors conducted an *in vitro* test for the purpose of selecting lactic acid bacteria having high skin improvement effect and treatment effect from those used in preparation of fermented milk, and thereby found that *Streptococcus thermophilus* OLS3059 has particularly high skin improvement and/or treatment effect, and the present invention was arrived at.

That is, the invention according to claim 1 is use of *Streptococcus thermophilus* OLS3059 in skin improvement and/or treatment.

The invention according to claim 2 is a composition for skin improvement and/or treatment, which comprises *Streptococcus thermophilus* OLS3059 and/or a culture thereof.

The invention according to claim 3 is the composition for skin improvement and/or treatment according to claim 2, which further comprises *Lactobacillus delbrueckii* subspecies *bulgaricus* OLL1073R-1 and/or a culture thereof.

The invention according to claim 4 is food and drink comprising the composition of claim 2.

The invention according to claim 5 is fermented milk for skin improvement and/or treatment prepared by using *Lactobacillus delbrueckii* subspecies *bulgaricus* and *Streptococcus thermophilus* OLS3059.

The invention according to claim 6 is the fermented milk for skin improvement and/or treatment according to claim 5, which further comprises collagen peptide and/or ceramide.

The invention according to claim 7 is a process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, charging the resulting milk into a container, and fermenting it to form curd followed by cold-storage thereof.

The invention according to claim 8 is a process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, fermenting the resulting milk to form curd, disrupting the curd, and charging it into a container followed by cold-storage thereof.

The invention according to claim 9 is a process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, fermenting the milk to form curd, disrupting the curd, cooling it, and charging it into a container followed by cold-storage thereof.

In the present invention, the phrase "skin improvement effect and/or treatment effect" refers to an improvement effect on at least one state selected from skin gloss, firmness, dullness, fleck, sagging, perceptible pores of the skin, fine texture, wrinkle, degree of dryness, stickiness, transparency, redness, spreading of foundation cream on the skin, swelling, perceptible dark circles under eyes, eruption, moisture retention, and deposition of pigment.

The phrase "skin improvement and/or treatment" refers to improvement of at least one state selected from skin gloss, firmness, dullness, fleck, sagging, perceptible pores of the skin, fine texture, wrinkle, degree of dryness, stickiness, transparency, redness, spreading of foundation cream on the skin, swelling, perceptible dark circles under eyes, eruption, moisture retention, and deposition of pigment.

The phrase "skin improvement and/or treatment" includes amelioration of skin conditions caused by atopic dermatitis and various kinds of allergic dermatitis, as well as suppression of development of such dermatitis.

The phrase "for skin improvement and/or treatment" is intended for the product of the invention to be used for the purpose of exhibiting the skin improvement effect and/or treatment effect, and is particularly intended for it to be suitable for use in exhibiting the skin improvement effect and/or treatment effect.

In the present invention, *Lactobacillus delbrueckii* subspecies *bulgaricus* is used in combination with *Streptococcus thermophilus* OLS3059 and used as the starter in production of fermented milk, thereby allowing the product to contain all lactic acid bacteria having an ability to form curds, and for example, there are lactic acid bacteria obtained by isolation from plain yogurt, hard yogurt and soft yogurt manufactured by Meiji Dairies Corporation.

In the present invention, *Streptococcus thermophilus* OLS3059 has been deposited under Accession No. FERM P-15487 (indication for identification: *Streptococcus thermophilus* OLS3059) since February 29, 1996 (accession date), with International Patent Organism Depositary (IPOD), National Institute of Advanced Industrial Science and Technology (AIST), Japan.

Transfer of this original deposition to deposition based on the Budapest treaty was conducted on November 29, 2006, under Accession No. FERM BP-10740 with the International Depository Authority.

The bacterial state of this *Streptococcus thermophilus* OLS3059 is as follows:
Colony form: cylindrical, trapezoidal and smooth.
(BL plate (horse blood-free), culture temperature: 37°C, culture time: 48 h)
Bacterial form: coccoid
Gram staining: positive
Oxygen requirement: aerobic
Sugar assimilability
Ribose -
Mannitol -
Sorbitol -
Lactose +
Trehalose -
Raffinose -
Maltose -
Melibiose -
Melezitose -
Arabinose -

In the present invention, *Lactobacillus delbrueckii* subspecies *bulgaricus* OLL1073R-1 has been deposited under Accession No. FERM P-17227 (indication for identification: *Lactobacillus delbrueckii* subspecies *bulgaricus* OLL1073R-1) since February 29, 1999 (accession date), with International Patent Organism Depositary (IPOD), National Institute of Advanced Industrial Science and Technology (AIST), Japan.

Transfer of this original deposition to deposition based on the Budapest treaty was conducted on November 29, 2006, under Accession No. FERM BP-10741 with the International Depository Authority. The bacterial state of *Lactobacillus delbrueckii* subspecies *bulgaricus* OLL1073R-1 is as follows:
Colony form: opaque and rough.
(BL agar plate, culture temperature: 37°C, culture time: 48 h)
Bacterial form: rod-shaped
Gram staining: positive
Oxygen requirement: facultative anaerobic
Sugar assimilability
Ribose -
Mannitol -
Sorbitol -
Lactose +
Trehalose -
Raffinose -
Maltose -
Melibiose -
Melezitose -
Arabinose -

### Effect of the Invention

The present invention can provide use of *Streptococcus thermophilus* OLS3059 in skin improvement and/or treatment.

According to the present invention, there can be provided a composition which is derived from natural products, is highly safe and has high skin improvement effect and/or treatment effect, a composition suitable for use in skin improvement and/or treatment, food and drink and fermented milk having a skin high improvement effect and/or treatment effect, comprising the composition, particularly a composition and fermented milk comprising lactic acid bacteria excellentin manufacturability.

According to the present invention, there can be provided fermented milk having a high skin improvement effect and treatment effect on humans for whom it exhibited a high intestinal function (bowel movement) improvement effect and/or treatment effect, as well as a process for producing the same.

### Best Mode for Carrying Out the Invention

The present invention provides use of *Streptococcus thermophilus* OLS3059 in skin improvement and/or treatment. That is, the present inventors made extensive study, and as a result, they found that that *Streptococcus thermophilus* OLS3059 has a particularly high skin improvement effect and/or treatment effect in an *in vitro* test conducted for the purpose of selecting lactic acid bacteria having a high skin improvement effect and treatment effect from those used in preparation of fermented milk, and the present invention was thereby completed.

Then, the composition of the present invention is a composition for skin improvement and/or treatment, which comprises *Streptococcus thermophilus* OLS3059 and/or a culture thereof. The composition for skin improvement and/or treatment according to the present invention is derived from natural products, and is thus highly safe and suitable for these applications.

The present inventors, then conducted an *in vitro* test for the purpose of selecting lactic acid bacteria having a high skinimprovementeffectand/ortreatmenteffectfromthose having an ability to form curds and being excellent in productivity and usable in preparation of fermented milk, found that *Streptococcus thermophilus* OLS3059 has a particularly high skin improvement effect and/or treatment effect as described above.

The composition for skin improvement and/or treatment comprising *Streptococcus thermophilus* OLS3059 and/or a culture thereof according to the present invention can be a composition further comprising *Lactobacillus delbrueckii* subspecies *bulgaricus* OLL1073R-1 and/or a culture thereof.

In the inventors' experiments, it was recognized that *Streptococcus thermophilus* OLS3059 and/or a culture thereof, when used in combination with *Lactobacillus delbrueckii* subspecies *bulgaricus* OLL1073R-1 extracellularly producing viscous polysaccharides, can also have a high skin improvement effect and/or treatment effect and is suitable for these applications.

*Streptococcus thermophilus* OLS3059 is used in, for example, Bulgarian yogurt LB81 (registered trademark) manufactured by Meiji Dairies Corporation.

*Streptococcus thermophilus* OLS3059 and/or a culture thereof, *Lactobacillus delbrueckii* subspecies *bulgaricus* OLL1073R-1 and/or a culture thereof, include bacteria themselves obtained by isolation and purification of the culture, the culture containing the bacteria, and any materials obtained by treating the culture, including an extract of the bacteria, a supernatant of the culture, a concentrate thereof, a concentrated or dried material thereof, and if necessary, a diluted liquid or material thereof, etc.

The above-mentioned *thermophilus* OLS3059 whether viable bacteria or killed bacteria has a high skin improvement effect and/or treatment effect and can be used in skin improvement and/or treatment. The above-mentioned *bulgaricus* OLL1073R-1 can also be used regardless of whether it is viable bacteria or killed bacteria. For *thermophilus* OLS3059 and *bulgaricus* OLL1073R-1, the culture method, extraction method, separation method, concentration method, drying method, dilution method etc. are notparticularlylimited. The medium for culturing the bacteria usually contains skim milk, whey, milk protein such as casein, sugars, yeast extract etc., and as the culture method, a wide variety of general aerobic or anaerobic methods can be used depending on the case. It is also possible to use a neutralization culture method wherein the culture temperature is set for example at 35 to 45°C, and the medium pH is kept in the neutral to acidic range, for example in the range of about pH 5 to 6 by using an alkali such as sodium hydroxide during culture. Besides the neutralization culture method, an arbitrary culture method such as a batch culture method can be used, and after culture, a culture or its supernatant can be subjected if necessary to concentration, drying, dilution etc. The culture may be separated by centrifugation or through a membrane into a supernatant and the bacteria, and the bacteria can also be recovered in a concentrated state. Then, the bacteria are subjected to sonication or enzyme treatment, whereby bacterial components can be extracted, or the culture, its supernatant, the bacteria, their extract etc. can be dried. These can be used as the active ingredient in the composition of the present invention.

The composition of the present invention can be compounded with an arbitrary component and used as an external preparation in a usual manner. For example, the composition can be blended if necessary with various components used in usual cosmetics, medicated cosmetics and pharmaceutical preparations and with an oil solution, a thickener, a preservative, an emulsifying agent, a pigment, a pH adjusting agent, an antioxidant and a perfume and used arbitrarily as cream, an emulsion, cosmetics, a foundation, a lotion and a shampoo depending on the object.

The food and drink of the present invention contain the composition of the present invention. The drink and food of the present invention has a high skin improvement effect and/or treatment effect and is suitable for these applications.

Because the active ingredient in the composition of the present invention is lactic acid bacteria which have been actually used in food, the composition is problematic with respect to safety and can be administered orally or parenterally as food, drink and a pharmaceutical preparation.

In this case, *Streptococcus thermophilus* OLS3059 can be used alone or as a mixture with usual human-diet components. Each of the *thermophilus* OLS3059 or *bulgaricus* OLL1073R-1 can be used alone, or these may be combined and used as a mixture with usual human-diet components. Alternatively, the *thermophilus* OLS3059, or a combination of *thermophilus* OLS3059 and *bulgaricus* OLL1073R-1, can be formed into a pharmaceutical preparation by mixing it with a wide variety of usual auxiliary substances used in production of an oral pharmaceutical preparation and/or a parenteral pharmaceutical preparation.

When *Streptococcus thermophilus* OLS3059, or a combination of *thermophilus* OLS3059 and *bulgaricus* OLL1073R-1, is used as a mixture with usual human-diet components or with a wide variety of usual auxiliary substances used in production of an oral pharmaceutical preparation and/or a parenteral pharmaceutical preparation, the (blending) amount of *thermophilus* OLS3059 can be determined suitably in consideration of the administration method and the age and weight of a person who ingests the food and drink of the invention, and is at least 10⁹ cfu/day, preferably at least 10¹⁰ cfu/day.

The food and drink of the present invention include those containing the composition of the present invention described above, which include not only drink and tablets but also biscuits, bread, sponge cakes, fermented milk, yogurt, cheese, butter, margarine, cream and jelly. With respect to the form and method in which the food and drink of the present invention are used, there are the form and method in which the composition of the present invention is added directly to the food and drink described above. In an alternative form and method, the yogurt, cheese, butter etc. that are the food and drink of the invention thus obtained are further added to food and drink.

For the purpose of exhibiting the skin improvement effect and/or treatment effect, the food and drink of the present invention can be used as usual food and drink but also as food for specified health use, nutrient functional food, health food etc.

The drink and food of the present invention have a skin improvement effect and/or treatment effect and can thus be prepared as food and drink provided with an indication to the effect that it is used for skin improvement and/or treatment. The method of indication includes description on a pamphlet, advertisement, etc., in addition to indication by a package for the food and drink.

The composition or the food and drink according to the present invention, when used as a pharmaceutical preparation, can be used in the form of tablets, capsules, granules, powder or syrup. In this case, composition or the food and drink can be formed into a pharmaceutical preparation by using usual auxiliary substances in addition to an excipient (that is, a base), a binder, a disintegrating agent, a lubricant, a flavoring substance, a smell corrective, a solubilizing agent, a suspending agent, and a coating agent.

The fermented milk of the present invention is fermented milk for skin improvement and/or treatment, which is prepared by using *Lactobacillus delbrueckii* subspecies *bulgaricus* and *Streptococcus thermophilus* OLS3059.

The fermented milk for skin improvement and/or treatment according to the present invention has a high skin improvement effect and/or treatment effect because it is prepared by using *Streptococcus thermophilus* OLS3059 having a high skin improvement effect and/or treatment effect from lactic acid bacteria having an ability to form curds, being excellent in productivity and usable in preparation of fermented milk. The fermented milk of the present invention has a high skin improvement effect and/or treatment effect and is suitable for these applications.

The above-mentioned *thermophilus* OLS3059 is combined with the *bulgaricus* bacteria to regulate lactic acid bacteria; for example, these are combined and used as the starter, whereby curds can be formed in a relatively short time.

For example, lactic acid bacteria obtained by isolation from plain yogurt, hard yogurt and soft yogurt manufactured by Meiji Dairies Corporation can be used as *Lactobacillus delbrueckii* subspecies *bulgaricus* in the fermented milk for skin improvement and/or treatment according to the present invention. The fermented milk of the present invention prepared by using a combination of these *bulgaricus* bacteria and *thermophilus* bacteria is characterized by being excellent in taste, having good flavor and smooth feeling on the tongue, and forming strong curds to be hardly broken during transportation of the product.

The fermented milk for skin improvement and/or treatment according to the present invention, prepared by using the above-mentioned *Lactobacillus delbrueckii* subspecies *bulgaricus* and *Streptococcus thermophilus* OLS3059, can further contain collagen peptide and/or ceramide.

When the collagen peptide- and ceramide-containing fermented milk of the invention is compared with the collagen peptide- and ceramide-free fermented milk of the invention, as will be described later, the collagen peptide- and ceramide-containing fermented milk tended to have a higher skin improvement effect and/or treatment effect. That is, the skin improvement effect and/or treatment effect attained by the fermented milk of the invention prepared by using the *bulgaricus* bacteria and *thermophilus* OLS3059 only was lower than that attained by the fermented milk of the invention prepared by using the *bulgaricus* bacteria and *thermophilus* OLS3059 having collagen peptide and/or ceramide further mixed therewith. This higher effect is considered attributable to the synergistic effect of mixing collagen peptide and/or ceramide with the lactic acid bacteria having a high skin improvement effect and/or treatment effect *(Streptococcus thermophilus* OLS3059).

From the viewpoint of exhibiting such synergistic effect, the collagen peptide contained in the fermented milk is preferably 500 mg% or more, more preferably 700 mg% or more, still more preferably 900 mg% or more. From the viewpoint of exhibiting the effect, the ceramide contained in the fermented milk is preferably 100 µg% or more, more preferably 200 µg% or more, still more preferably 250 µg% or more.

From the viewpoint of the synergistic effect for skin improvement effect and/or treatment effect, it is preferable to add, to the fermented milk of the present invention, not only collagen peptide and/or ceramide but also various active ingredients used in foods, drinks and pharmaceutical preparations for metabolic improvement, such as sphingomyelin, isoflavone, chondroitin and hyaluronic acid.

To find a factor influencing the skin improvement effect and/or treatment effect of the fermented milk for skin improvement and/or treatment according to the present invention, the inventors made further detailed examination in a human test, and as a result, they found that the fermentedmilk of the invention described above has a higher skin improvement effect and/or treatment effect on humans for whom it shows an improvement effect on regulation of intestinal functions, which is regarded as an action typical of fermented milk.

Specifically, the collagen- and ceramide-containing fermented milk for skin improvement and/or treatment according to the present invention and the collagen- and ceramide-free fermented milk for skin improvement effect and/or treatment according to the present invention were used and compared with each other in respect of the improvement effect on regulation of intestinal functions and the improvement effect on the skin, thereby attaining the finding described above.

That is, the fermented milk for skin improvement and/or treatment according to the present invention is **characterized in that** it exhibits a higher skin improvement effect and/or treatment effect on humans for whom it exhibits a high intestinal function regulation (bowel movement) improvement effect and/or treatment effect.

The process for producing fermented milk according to the present invention wherein the fermented milk for skin improvement and/or treatment according to the present invention is produced, comprises:
preparing raw milk, adding *Lactobacillus delbrueckii* subspecies *bulgaricus* and *Streptococcus thermophilus* OLS3059 as starters to the milk, charging the resulting milk into a container, and fermenting it to form curd followed by cold-storage thereof.

Alternatively, the process comprises preparing raw milk, adding *Lactobacillus delbrueckii* subspecies *bulgaricus* and *Streptococcus thermophilus* OLS3059 as starters to the milk, fermenting the resulting milk to form curd, disrupting the curd, and charging it into a container followed by cold-storage thereof;
or the process comprises preparing raw milk, adding *Lactobacillus delbrueckii* subspecies *bulgaricus* and *Streptococcus thermophilus* OLS3059 as starters to the milk, fermenting the milk to form curd, disrupting the curd, cooling it, and charging it into a container followed by cold-storage thereof.

Inpreparation of rawmilkbefore addition of *Lactobacillus delbrueckii* subspecies *bulgaricus* and *Streptococcus thermophilus* OLS3059 as the starter to the milk, the raw milk is prepared and subjected to treatments such as homogenization, sterilization and cooling in the same manner as in a conventional process of producing fermented milk.

In the process for producing the fermented milk of the present invention, *Streptococcus thermophilus* OLS3059 having a high skin improvement effect and/or treatment effect, selected from lactic acid bacteria having an ability to form curds, being excellent in productivity and being used in production of fermented milk, is combined with the *bulgaricus* bacteria and used as the starter.

Accordingly, the problem of the conventional lactic acid bacteria having a skin improvement effect etc. has been solved. Conventionally, lactic acid bacteria having an improvement effect on skin etc. but poor in manufacturability of fermented milk, and lactic acid bacteria excellent in manufacturability for forming curds, are separately managed and the fermentation time is prolonged, so there is a problem that the manufacturing time is increased as a whole. That is, conventional lactic acid bacteria having an improvement effect on skin etc. are not suitable for large-scale production (commercial production) of fermented milk etc.

However, the process for producing the fermented milk according to the present invention has solved the problem described above and can thus be applied to commercial production. It is important for commercial production that the process is simple with a fewer number of steps.

In the process for producing the fermented milk of the present invention, homogenization in the process for producing raw milk can be carried out before and/or after sterilization.

As described above, cooling after fermentation may be carried out after formation of curd or disruption of curd, or after filling in a container.

Disruption of curd can be followed by adding or mixing fruit flesh, vegetables, various sauces and/or various additives.

In the process for producing the fermented milk of the present invention, the fermentation time is 8 hours or less, preferably 6 or 5 hours or less, more preferably 4 hours or less, in such a range as to be usable in usual production of fermented milk.

The fermented milk for skin improvement and/or treatment according to the present invention can be regularly used and easily and successively ingested owing to its extremely excellent flavor. The skin improvement effect and treatment effect can thereby be further increased.

Hereinafter, the present invention is described by reference to preferable examples, but the present invention is not limited thereto.

### Example 1

### (Verification of inhibitory effect on atopic dermatitis in an in vitvo test)

Four-week-old female NC/Nga mice (manufactured by Nippon SLC Co., Ltd.) were acclimated and raised and then divided into the following 4 groups:
a distilled water administration group (n = 10),
a *Streptococcus thermophilus* OLS3059 (also referred to hereinafter as OLS3059; isolated from Bulgarian yogurt LB81 (registered trademark) manufactured by Meiji Dairies Corporation) administration group (n = 10),
a dermatitis non-induction group (n = 7),
and a *Lactobacillus rhamnosus* ATCC 53103 (also referred to hereinafter as ATCC 53103) administration group (n = 10) as a control for comparison with the OLS3059 administration group.

Feed (trade name: CRF-1, manufactured by Oriental Yeast Co., Ltd.) and distilled water were freely given to the mice.

ATCC 53103 is a lactic acid bacterium known to be effective against atopic dermatitis.

During the test period, distilled water or each lactic acid bacterium was orally administered with a probe to the 3 groups excluding the dermatitis non-induction group. According to a method described later, induction of dermatitis was conducted every day from Day 0 (that is, 7 days after the test was initiated). That is, for the test period (from the 1st day (= Day -7) to the 21st day (= Day 14)), distilled water was administered orally to the stomach of the distilled water administration group every day (1 mg/day/mouse) and an aqueous suspension of each kind of lactic acidbacteriumwas administered in the same manner to the stomach of the lactic acid bacterium administration group (1 mg/day/mouse). The suspension of each kind of lactic acid bacterium was prepared by suspending a lyophilized product of the bacterium subjected to heat treatment (temperature 75°C for 60 minutes). In the suspension of each kind of lactic acid bacterium, the density of bacteria was 7.13×10⁸ cfu/g for ATCC 53103 or 8.56×10⁷ cfu/g for OLS3059.

Dermatitis was induced from 7 days (Day 0) after the test was initiated, and a change in development was scored. Serum was collected 15 days (Day 15) after the dermatitis was induced, and the IgE level was measured by the ELISA method. The IgE level was measured by using an antibody (manufactured by Pharmingen) in accordance with a modification to Pharmingen' recommended protocol.

A disrupted mite liquid was prepared in the following manner. That is, the whole of a mite *Dermatophagoides pteronyssinus* (Mite-Dp, manufactured by LSL) was defatted with anhydrous ether, then distilled water was added thereto, and the mite was disrupted by sonication. Then, the resulting sample was centrifuged to give a water-soluble fraction which was then lyophilized. This lyophilized sample was dissolved in distilled water to prepare a solution with a protein concentration of 4.5 mg/ml (in terms of BSA, determined by DC protein assay from BIO-RAD).

The dermatitis induction test was carried out using a modification to the method of Kaino et al. (Tetsushi Kaino et al., Allergy 50:1152-1162, 2001). A dermatitis induction site (head, auricle and cervical region) was dehaired with a hair clipper, and an aqueous solution of SDS (4%) was applied 20 times with a brush onto the dermatitis induction site of every mouse. The amount of the SDS solution thus applied 20 times was about 60 µl in total. After the aqueous solution of SDS was dried, the disrupted mite liquid and distilled water were applied 20 times in the same manner to the dermatitis induction group and the dermatitis non-induction group, respectively. The above-described operation was repeated every day as one set thereby inducing dermatitis and repeatedly carried out on consecutive days from 7 days (Day 0) after the test was initiated to the day when the test was terminated.

Measurement of IgE was carried out in the following method. That is, an anti-IgE antibody for primary antibody (manufactured by Pharmingen) was added at 2 µg/ml to a 96-well plate and left for 1 hour at a temperature of 37°C. After washing with Tween 20/PBS (PBS-Tween, 0.05%), BSA/PBS (1%) was added and left at room temperature for 30 minutes. After washing with PBS-Tween, a serum sample and IgE for preparation of standard curve (manufactured by Pharmingen) were added and left at room temperature for 30 minutes. After washing with PBS-Tween, biotin-anti-IgE antibody for secondary antibody (manufactured by Pharmingen) was added at 0.5 µg/ml and left at room temperature for 1 hour. After washing with PBS-Tween, streptoavidin-HRP (manufactured by Pharmingen) was added and left at room temperature for 30 minutes. After washing with PBS-Tween, TMB + Substrate Chromogen (manufactured by DAKO) was added to initiate coloration reaction. After the coloration reaction, sulfuric acid (1 N) was added, and the absorbance (450 nm) was measured with a microtiter plate, and the IgE level in serum was calculated.

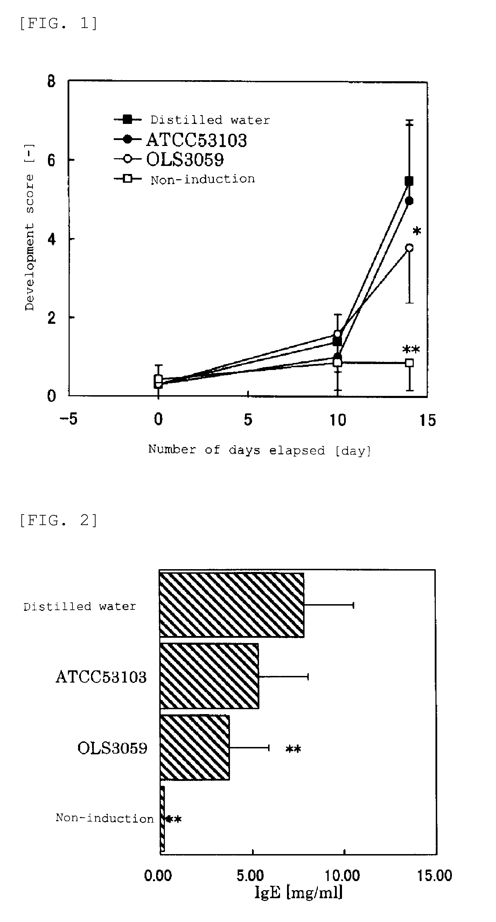

The change in dermatitis development score is shown in FIG. 1. The 5 items: [1] dry skin and crust formation, [2] reddening and bleeding, [3] tissue dropping and scratch, [4] edema, and [5] itching behavior were scored in 4 ranks [0. no symptoms], [2. light], [3. moderate] and [4. severe] to calculate the total score in evaluation of each mouse. The development score 13 days (Day 13) after induction of dermatitis had been initiated was significantly lower in the OLS3059 administration group than in the distilled water administration group. On the other hand, the development score in the ATCC 53103 administration group was not significantly different from that in the distilled water administration group.

The total IgE level is shown in FIG. 2. The total IgE in serum 15 days (Day 15) after induction of dermatitis had been initiated was significantly lower in the OLS3059 administration group than in the distilled water administration group. The total IgE in serum in the ATCC 53103 administration group was slightly lower than that in the distilled water administration group, but a significant difference therebetween was not recognized. ATCC 53103 is a lactic acid bacterium known to be effective against atopic dermatitis, but OLS3059 had a higher inhibitory effect on development of atopic dermatitis than by ATCC 53103.

### Example 2

### (Verification of inhibitory effect on atopic dermatitis in an in vivo test)

Four-week-old female NC/Nga mice (manufactured by Nippon SLC Co., Ltd.) were acclimated and raised and then divided into the following 3 groups:
a distilled water administration group (n = 4),
a *Lactobacillus delbrueckii* subspecies *bulgaricus* OLL1073R-1 (also referred to hereinafter as OLL1073R-1) administration group (n = 6),
and a *Streptococcus thermophilus* OLS3059 (isolated from Bulgarian yogurt LB81 (registered trademark) manufactured by Meiji Dairies Corporation) administration group (n = 5).

Feed (trade name: CRF-1, manufactured by Oriental Yeast Co., Ltd.) and distilled water were freely given to the mice. The experimental conditions were the same as in Example 1 except that the period of induction of dermatitis was 30 days.

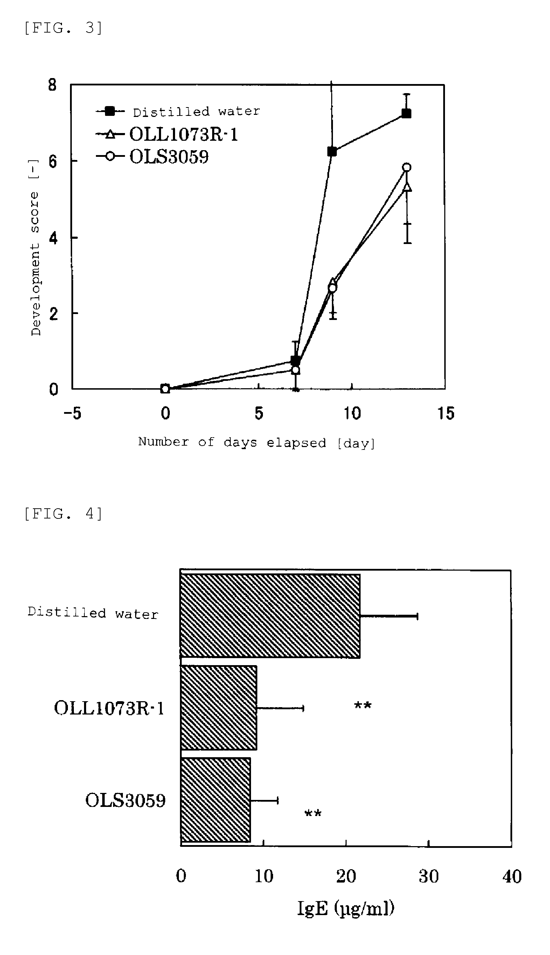

The change in dermatitis development score is shown in FIG. 3. The development score 13 days (Day 13) after induction of dermatitis had been initiated was significantly lower in the OLL1073R-1 group and the OLS3059 administration group than in the distilled water administration group.

The total IgE level is shown in FIG. 4. The total IgE in the OLL1073R-1 group and in the OLS3059 administration group was significantly lower than in the distilled water administration group. The OLS3059 of the present invention was recognized to have an inhibitory effect on development of atopic dermatitis. The OLL1073R-1 of the present invention was also recognized to have an inhibitory effect on development of atopic dermatitis, but the IgE level was lower in the OLS3059 of the present invention.

### Example 3

### (Verification of inhibitory effect on atopic dermatitis in an in vivo test)

The OLL1073R-1 of the present invention, which had been confirmed in Example 2 to exhibit an inhibitory effect comparable to that of the OLS3059 of the present invention on development of atopic dermatitis, was used in combination with the OLS3059 of the present invention, to examine the inhibitory effect thereof on development of atopic dermatitis.

As a control for comparison with the combination of OLS3059 and OLL1073R-1 in the present invention, a combination of *Lactobacillus delbrueckii* subspecies *bulgaricus* MEP1705601 and *Streptococcus thermophilus* MEP1705601, both of which were isolated from Bulgarian yogurt (manufactured by Meiji Dairies Corporation), was used.

Four-week-old female NC/Nga mice (manufactured by Nippon SLC Co., Ltd.) were acclimated and raised and then divided into the following 4 groups.

That is, the mice were classified into a group given a fermented product produced using *Lactobacillus delbrueckii* subspecies *bulgaricus* OLL1073R-1 and *Streptococcus thermophilus* OLS3059 (also referred to hereinafter as OLL1073R-1 + OLS3059 administration group) (n = 8),
a group given a fermented product produced using *Lactobacillus delbrueckii* subspecies *bulgaricus* MEP1705601 and *Streptococcus thermophilus* MEP1705601 (also referred to hereinafter as LB MEP1705601 + ST MEP1705601 administration group) (n = 9),
a non-fermented product administration group (n = 9) and a dermatitis non-induction, non-fermented product administration group (also referred to hereinafter as non-induction group) (n = 4).

Either the fermented product or the non-fermented product was ingested in an amount of 100 mg/mouse/day, and orally administered on consecutive days until the day when the test was terminated.

The number of bacteria in the fermented product was 1×10⁹ cfu/g for OLL1073R-1, 3×10⁹ cfu/g for OLS3059, 8.6×10⁸ cfu/g for LB MEP1705601, and 2.1×10⁹ cfu/g for ST MEP1705601.

Each of the fermented product and the non-fermented product was prepared by lyophilization and suspended in distilled water before administration. The experimental conditions were the same as in Example 1 except that the dermatitis induction period was 29 days.

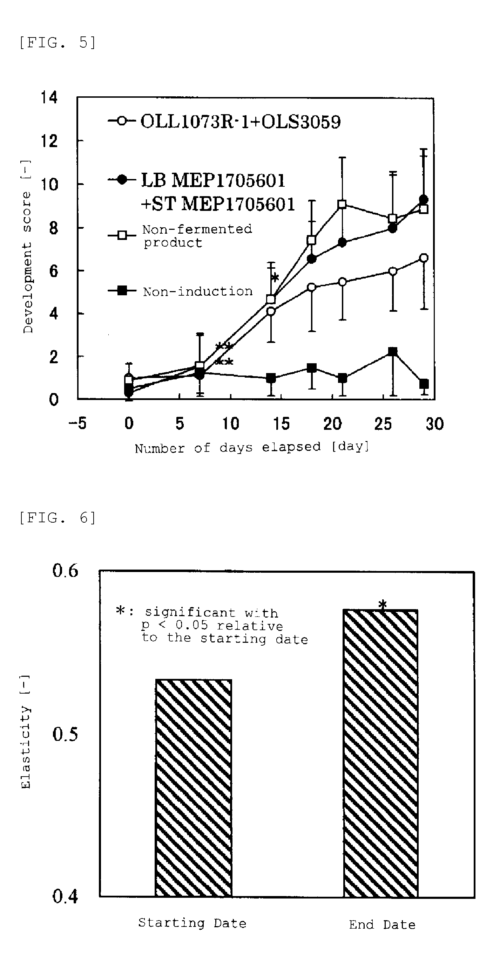

The change in dermatitis development score is shown in FIG. 5. The development score 14 days (Day 14) after induction of dermatitis had been initiated was significantly lower in the OLL1073R-1 + OLS3059 administration group than in the LB MEP1705601 + ST MEP1705601 administration group or in the non-fermented product administration group.

The development score on days following 14 days (Day 14) after induction of dermatitis had been initiated was significantly lower in the OLL1073R-1 + OLS3059 administration group than in the LB MEP1705601 + ST MEP1705601 administration group or in the non-fermented product administration group.

It was confirmed that OLL1073R-1, which hadbeen confirmed in Example 2 to exhibit an inhibitory effect comparable to that of the OLS3059 of the present invention on development of atopic dermatitis, can used in combination with the OLS3059 of the present invention, to exhibit an inhibitory effect on development of atopic dermatitis.

### Example 4

### (Fermented Milk Production Example 1)

50.0 g raw milk, 5.0 kg skim milk, 8.0 kg sugar, 1.0 kg collagen peptide (trade name: Nippi Peptide, manufactured by Nippi Collagen Cosmetics, Ltd.), 0.1 kg ceramide-containing konnyaku (mannan) potato extract (trade name: Konnyaku Ceramide, manufactured by Unitika Ltd.), 0.1 kg perfume, and 33.8 kg water were mixed and dissolved at a temperature of 65°C to prepare a milk preparation. This milk preparation was subjected to homogenization treatment at a temperature of 65°C (pressure 100 kg/cm²) and then sterilized at a temperature of 95°C for 2 minutes and immediately cooled to a temperature of 43 to 45°C. 2.0 kg lactic acid bacterium starter was added to this sterilized milk preparation which was then charged into a container, followed by fermentation at a temperature of 43°C for 4 hours to give fermented milk (set yogurt). The lactic acid bacterium starter used was a mixture of equal volumes of *Lactobacillus bulgaricus* 2038 isolated from Bulgarian yogurt LB81 (registered trademark) manufactured by Meiji Dairies Corporation and *Streptococcus thermophilus* OLS3059 (also referred to hereinafter as OLS3059, isolated from Bulgarian yogurt LB81 (registered trademark) manufactured by Meiji Dairies Corporation).

### Example 5

### (Fermented Milk Drink Production Example 1)

23.0 g raw milk, 12.1 kg skim milk and 62.9 kg water were mixed and dissolved at a temperature of 70°C to prepare a milk preparation. This milk preparation was subjected to homogenization treatment at a temperature of 65°C (pressure 150 kg/cm²) and then sterilized at a temperature of 95°C for 10 minutes and immediately cooled to a temperature of 40 to 45°C. 2.0 kg lactic acid bacterium starter was added to this sterilized milk preparation, followed by fermentation at a temperature of 40 to 45°C for 5 hours to give fermented milk.

The lactic acid bacterium starter used was a mixture of equal volumes of *Lactobacillus bulgaricus* 2038 isolated from Bulgarian yogurt LB81 (registered trademark) manufactured by Meiji Dairies Corporation and *Streptococcus thermophilus* OLS3059 (also referred to hereinafter as OLS3059, isolated from Bulgarian yogurt LB81 (registered trademark) manufactured by Meiji Dairies Corporation).

For mechanically breaking curds of this fermented milk, the fermented milk was subjected to homogenization treatment (pressure 150 kg/cm²) and then cooled to a temperature of 5°C to give a fermentedmilkpreparation. Separately, 3.0 kg pectin, 2.0 kg sugar, 0.02 kg sweetener, 0.1 kg perfume and 34.0 kg water were mixed and dissolved at a temperature of 65°C, then sterilized at a temperature of 130°C for 2 seconds, and immediately cooled to a temperature of 5°C to form a sugar solution.

The fermented milk preparation was mixed with the sugar solution at a predetermined concentration to give a fermented milk drink (drink-type yogurt).

### Example 6

### (Fermented Milk Drink Production Example 2)

23.0 g raw milk, 12.1 kg skim milk and 62.9 kg water were mixed and dissolved at a temperature of 70°C to prepare a milk preparation. This milk preparation was subjected to homogenization treatment at a temperature of 65°C (pressure 150 kg/cm²) and then sterilized at a temperature of 95°C for 10 minutes and immediately cooled to a temperature of 40 to 45°C. 2.0 kg lactic acid bacterium starter was added to this sterilized milk preparation, followed by fermentation at a temperature of 40 to 45°C for 5 hours to give fermented milk.

The lactic acid bacterium starter used was a mixture of equal volumes of *Lactobacillus bulgaricus* 2038 isolated from Bulgarian yogurt LB81 (registered trademark) manufactured by Meiji Dairies Corporation and *Streptococcus thermophilus* OLS3059 (also referred to hereinafter as OLS3059, isolated from Bulgarian yogurt LB81 (registered trademark) manufactured by Meiji Dairies Corporation).

For mechanically breaking curds of this fermented milk, the fermented milk was subjected to homogenization treatment (pressure 150 kg/cm²) and then cooled to a temperature of 5°C to give a fermentedmilkpreparation. Separately, 3.0 kg pectin, 2.0 kg sugar, 0.02 kg sweetener, 1.0 kg collagen peptide (trade name: Collagen Peptide 5200, manufactured by Nitta Gelatin Co. , Ltd.), 0.03 kg ceramide-containing corn extract (trade name: Corn Ceramide ME1-R, manufactured by Tsuji Seiyu Co., Ltd.), 0.1 kg perfume and 34.0 kg water were mixed and dissolved at a temperature of 65°C, then sterilized at a temperature of 130°C for 2 seconds, and immediately cooled to a temperature of 5°C to form a sugar solution.

The fermented milk preparation was mixed with the sugar solution at a predetermined concentration to give a fermented milk drink (drink-type yogurt).

### Example 7

### (Milk Drink Production Example)

1 g lyophilized powder of *Streptococcus thermophilus* OLS3059 (also referred to hereinafter as OLS3059, isolated from Bulgarian yogurt LB81 (registered trademark) manufactured by Meiji Dairies Corporation) was added to 1000 ml rawmilk to prepare a milk preparation. This milk preparation was subjected to homogenization treatment, then sterilized at a temperature of 130°C for 3 seconds and immediately cooled to a temperature of 10°C or less. This sterilized milk preparation was charged into a container to give a milk drink.

### Example 8

### (Verification of skin improvement in a human test)

The above fermented milk *(Streptococcus thermophilus* OLS3059; density of bacteria, 85×10⁷ cfu/g; collagen peptide, 1000 mg/90 g; ceramide, 300 µg/90 g) in Example 4 was used as a test meal in a human test where the test meal was ingested twice (that is, morning and night respectively) per day for 28 days in an amount of 90 g for each ingestion. The subjects were 31 women (average age: 31.8 years old).

On the starting date (before ingestion of the test meal) and on the end date (Day 28), the elasticity and texture density of the subjects' skin were evaluated and observed, and in addition, the skin was observed for eruptions by a physician. The skin elasticity was determined by selecting one of right-and-left cheeks and measuring one site twice on the cheek (trade name: CUTOMETER SEM575, manufactured by Courage+Khazaka). The texture density of the skin was determined by selecting a site of rough dry skin and measuring one site (trade name: Visual Imager VI-20, manufactured by Inforward, Inc.). Eruptions were evaluated by determining the total number of eruptions.

On the starting date (before ingestion of the test meal) and on the end date (Day 28), the subjects were surveyed by questionnaire. The questionnaire is shown in Table 1. As shown in Table1, the 18 items: [1] skingloss, [2] firmness, [3] dullness, [4] fleck, [5] sagging, [6] pores of the skin, [7] fine texture, [8] wrinkle (forehead), [9] wrinkle (around lips), [10] wrinkle (around eyes), [11] degree of dryness, [12] stickiness, [13] transparency, [14] redness, [15] spreading of foundation cream on the skin, [16] swelling, [17] constipation, and [18] dark circles under eyes, were evaluated in 5 ranks. On the end date, the usefulness of the test meal was evaluated by both the subject and a physician.

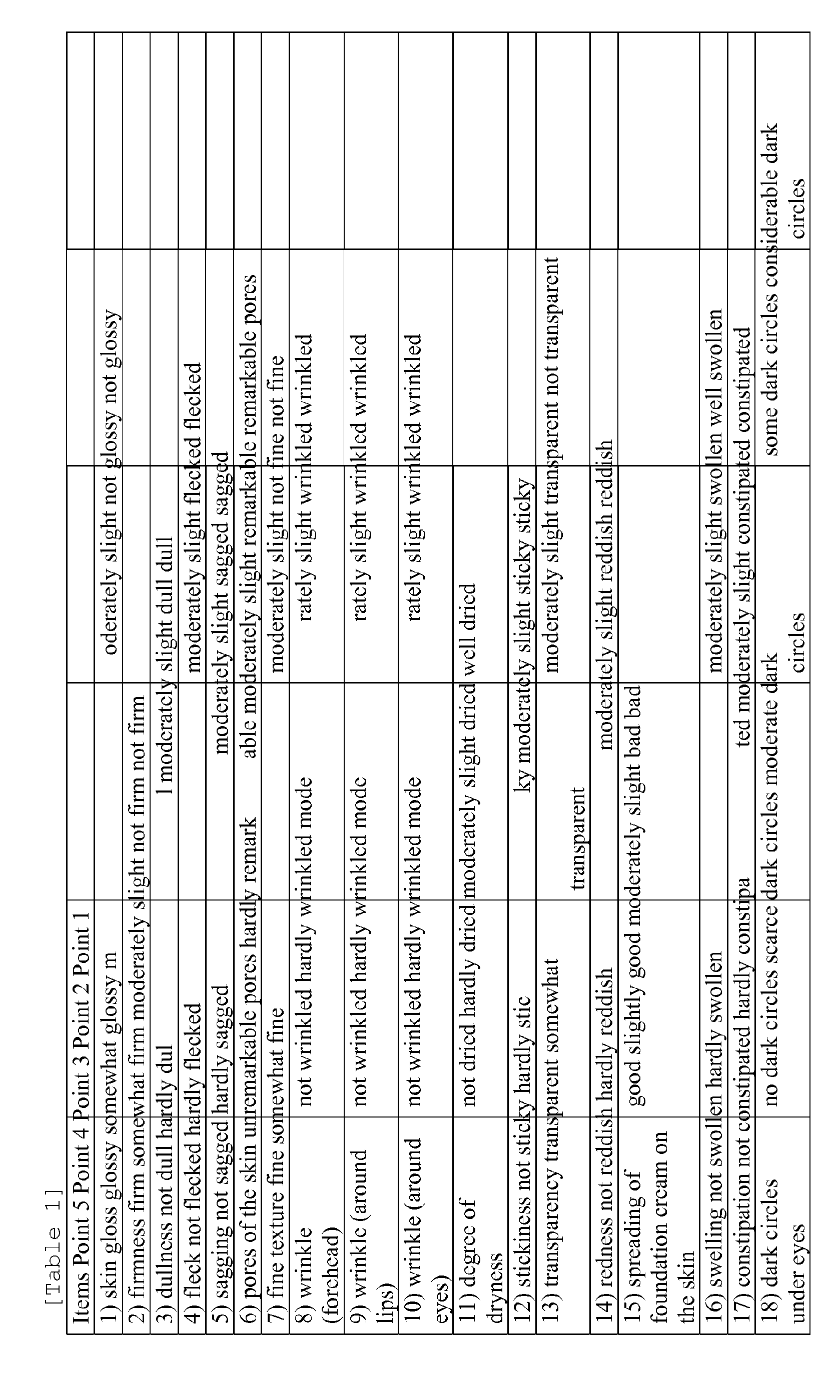

The evaluation result of skin elasticity upon administration of the test meal in Example 5 is shown in FIG. 6. By ingesting the test meal in Example 5, the skin elasticity was significantly increased, and the skin firmness was improved. The evaluation result of texture density of the skin upon administration of the test meal in Example 5 is shown in FIG. 7. By ingesting the test meal in Example 5, the texture density of the skin was significantly increased, and the texture density of the skin was improved. The evaluation result of total number of eruptions upon administration of the test meal in Example 5 is shown in FIG. 8. By ingesting the test meal in Example 5, the total number of eruptions was significantly decreased, and the rough surface was ameliorated.

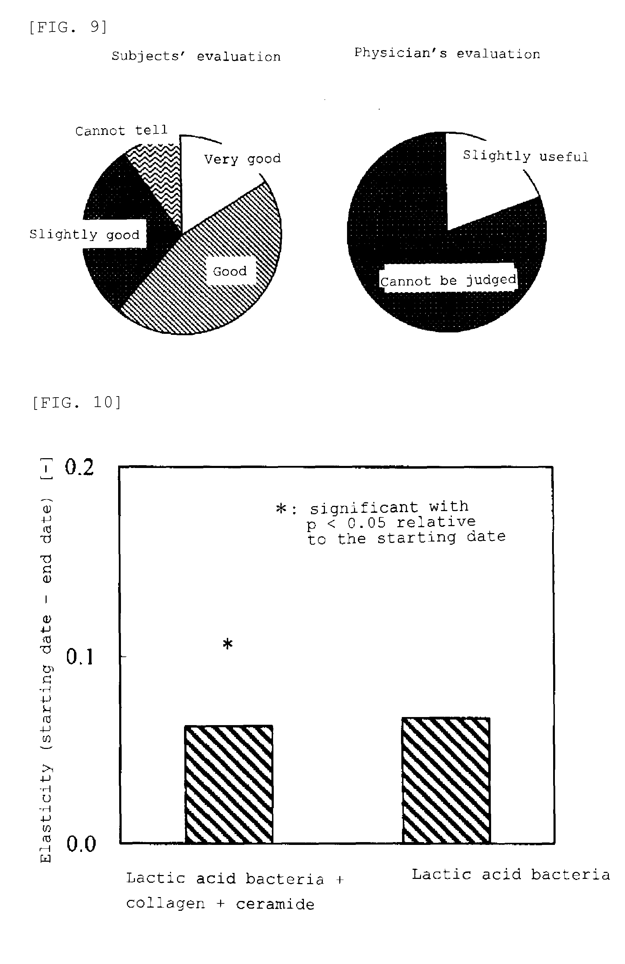

The result of questionnaire survey of the subjects is shown in Table 2. It was confirmed that the skin condition and constipation of the subjects are ameliorated by ingesting the test meal. The evaluation result of usefulness of the test meal by both the subject and physician is shown in FIG. 9. It was recognized by the subject or the physician that ingestion of the test meal is useful for the skin. The excellent skin improvement effect and/or treatment effect of *Streptococcus thermophilus* OLS3059 was demonstrated, and *Streptococcus thermophilus* OLS3059 was confirmed to be suitable for use in exhibiting the skin improvement effect and/or treatment effect.

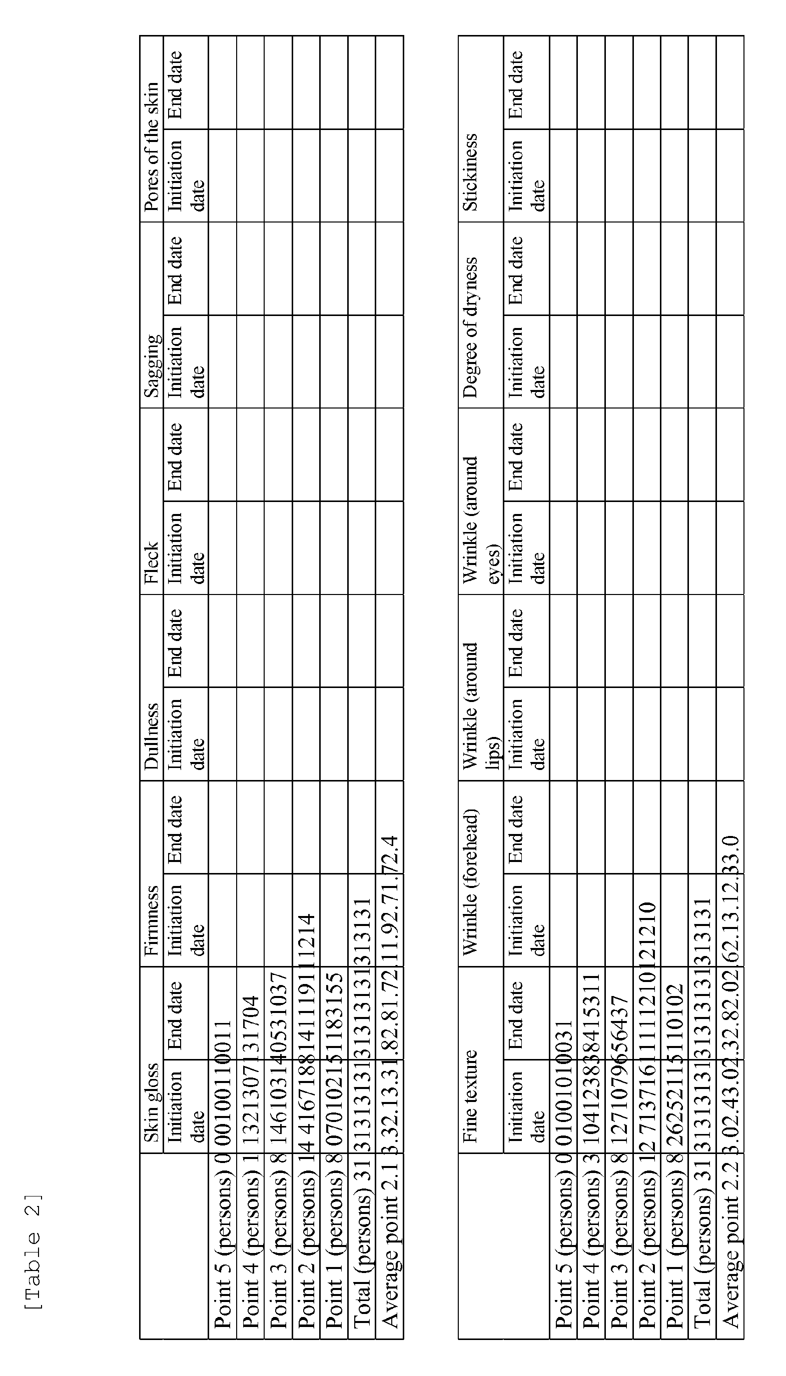

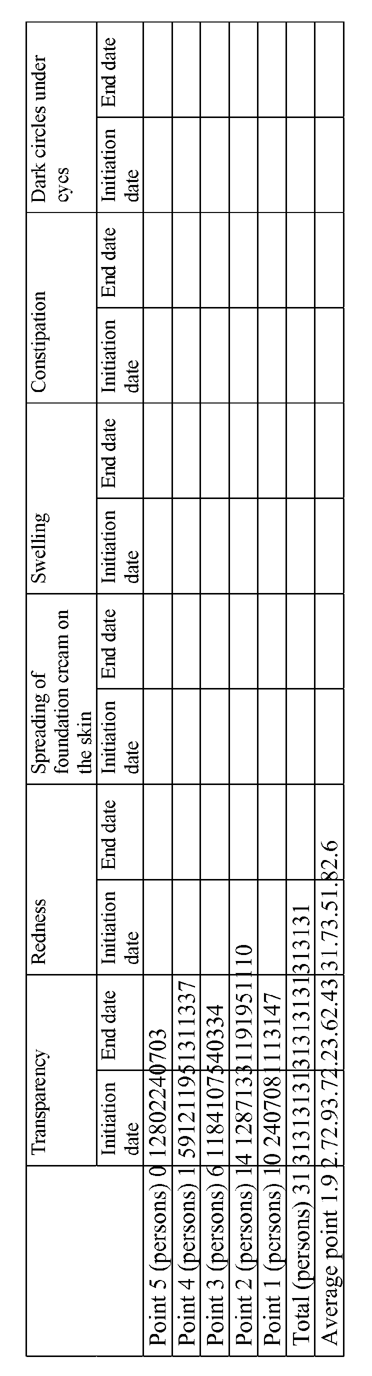

### Example 9

### (Verification of the relationship between high intestinal function regulation improvement and skin improvement effect in a human test)

The fermented milk *(Streptococcus thermophilus* OLS3059; density of bacteria, 50×10⁷ cfu/g) in Example 5 and the fermented milk *(Streptococcus thermophilus* OLS3059; density of bacteria, 50×10⁷ cfu/g; collagen peptide, 1000 mg/120 ml; ceramide, 300 µg/120 ml) in Example 6 were used as test meals in a human test where the test meals were ingested twice (morning and night respectively) per day for 28 days in an amount of 120 g for each ingestion. The fermented milk drink in Example 5 was examined in 28 women as subjects (average age: 31.1 years old), and the fermented milk drink in Example 6 was examined in 28 women as subjects (average age: 30.3 years old).

The questionnaire survey of the subjects was conducted in the same manner as in Example 8. The questionnaire is as shown in Table 1. The results of questionnaire survey of the subjects are shown in Tables 3 and 4. It was confirmed that the skin state and constipation of the subjects are ameliorated by ingesting the test meals. The test meal (containing collagen and ceramide) in Example 6 had a particularly higher effect than by the test meal in Example 5 on 6 items in Table 1: that is, [1] skin gloss, [2] firmness, [7] fine texture, [11] degree of dryness, [15] spreading of foundation cream on the skin, and [17] constipation.

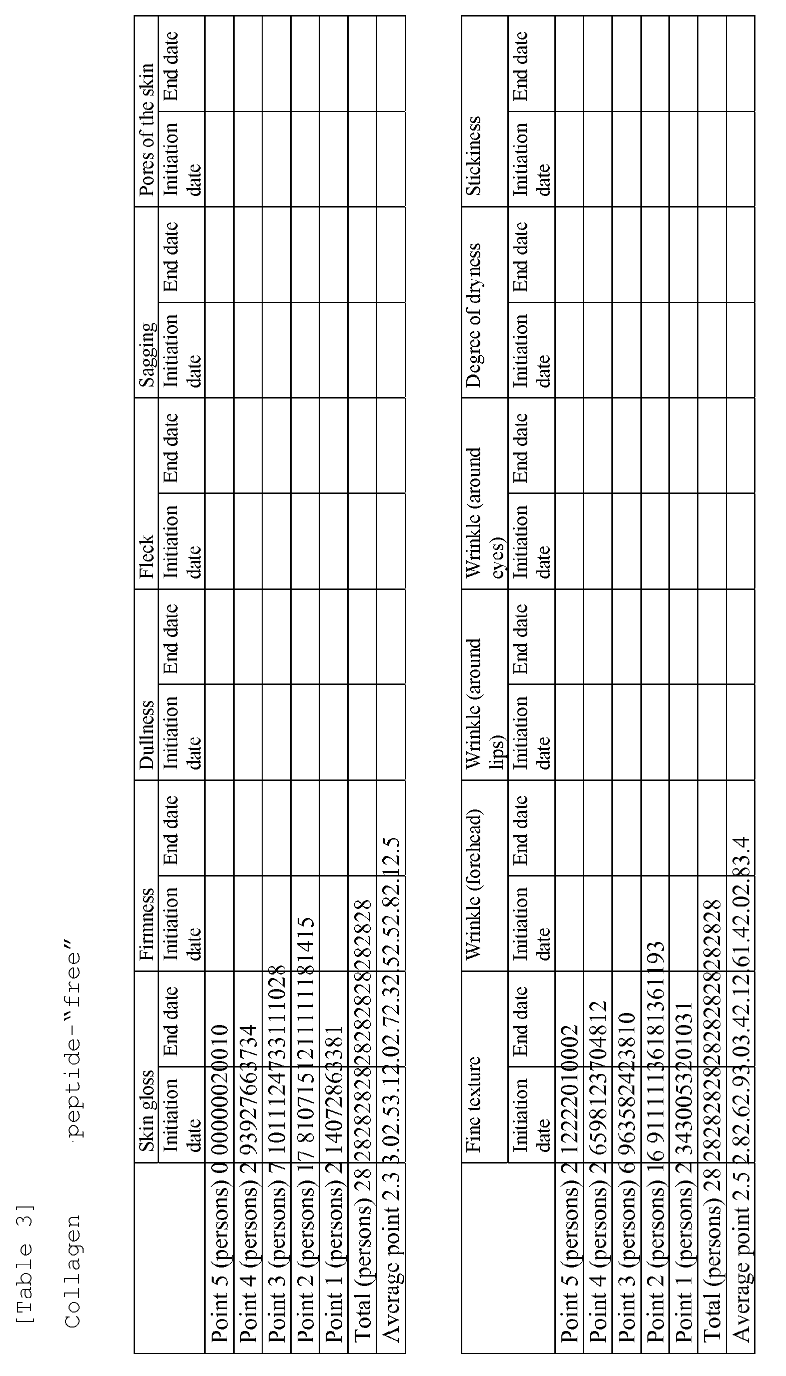

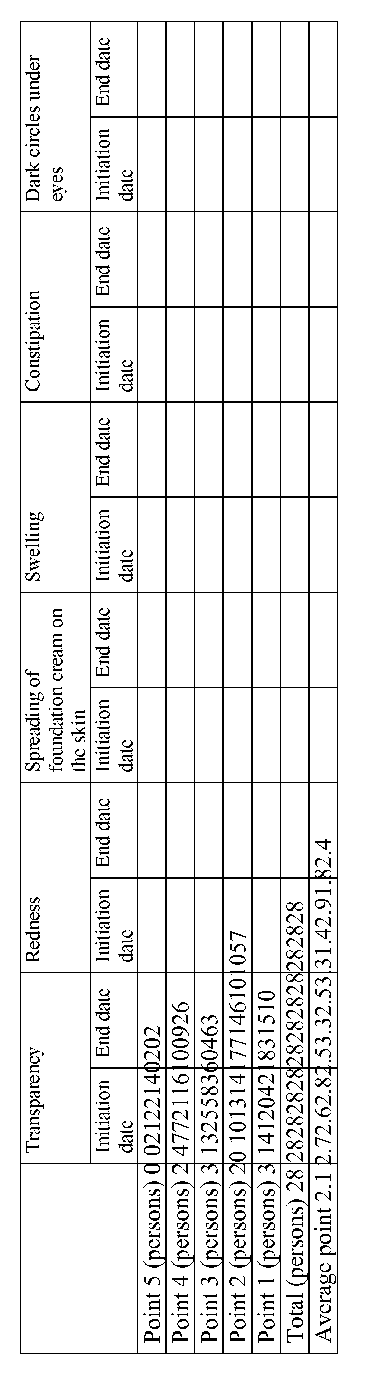

The change in intestinal function regulation (bowel movement) score is shown in Table 4. Scoring was conducted in the 5 ranks: "1. not constipated", "2. hardly constipated, " "3. moderate, " "4. slightly constipated," and "5. constipated," and the difference in each subject before ingestion and after ingestion was calculated and evaluated. For example, when the subject whose had been "5. constipated" before ingestion of the test meal became "3. moderate" after ingestion, the intestinal function regulation was analyzed as being improved by "2 or more" .

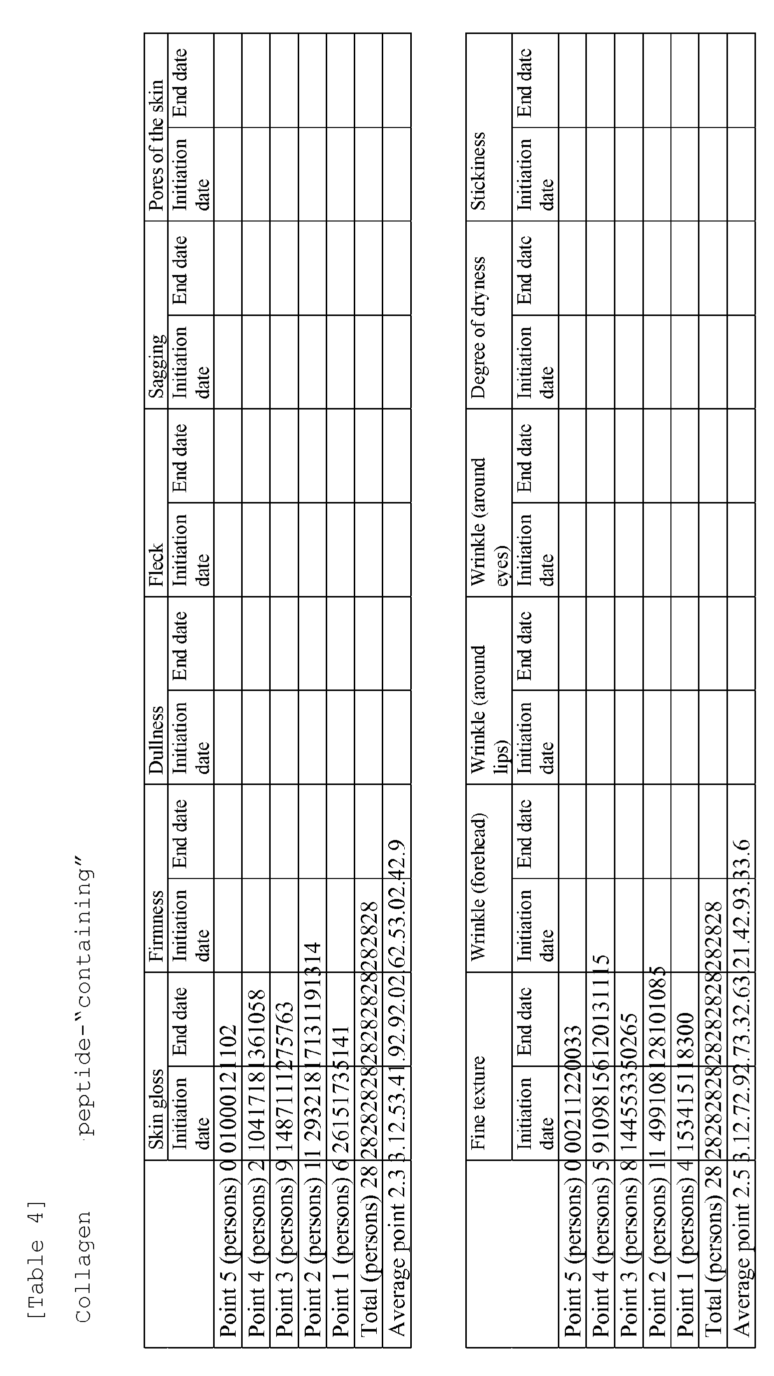

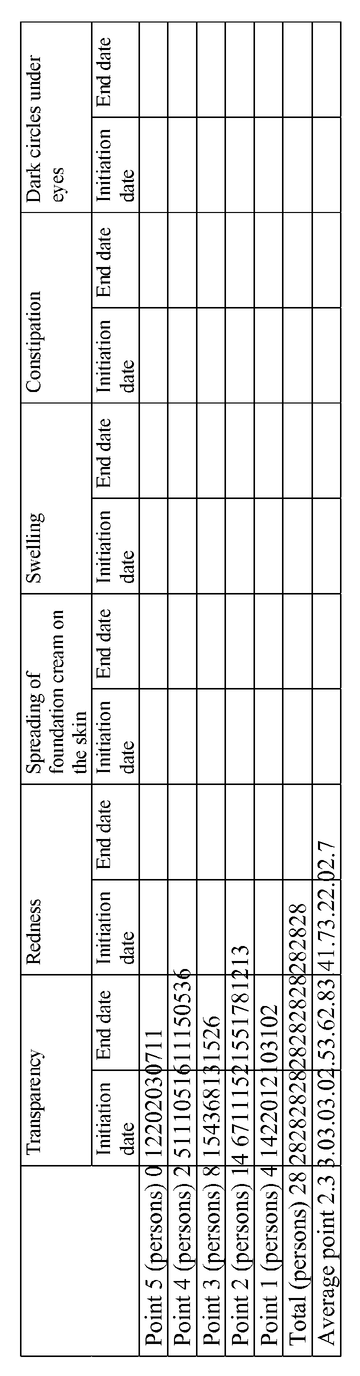

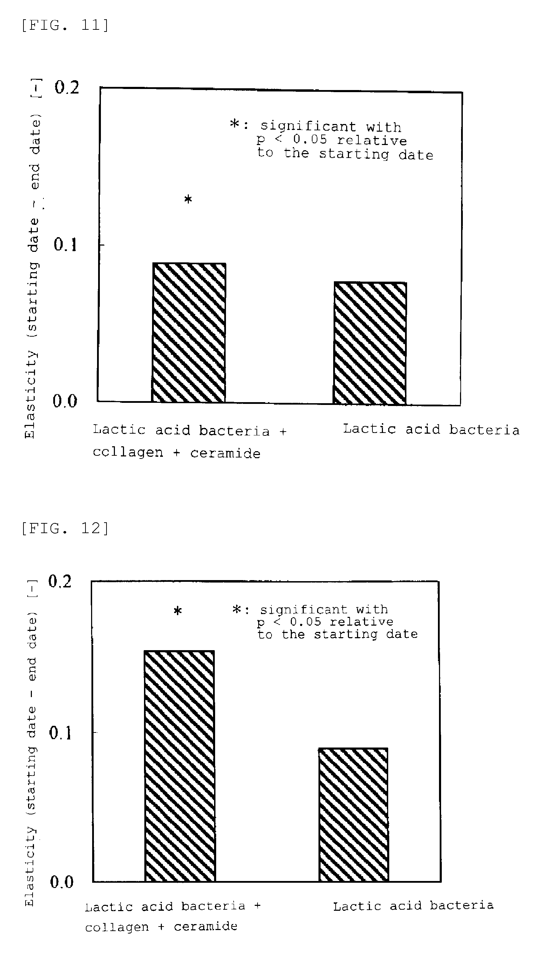

The results of evaluation of skin elasticity upon ingestion of the test meals in Examples 5 and 6 are shown in FIGS. 10 to 12. FIG. 10 shows the result of evaluation of the skin elasticity of the subject having intestinal function regulation improved by "1 or more", FIG. 11 shows the result of evaluation of the skin elasticity of the subject having intestinal function regulation improved by "2 or more", and FIG. 12 shows the result of evaluation of the skin elasticity of the subject having intestinal function regulation improved by "3 or more". It was demonstrated that for the human for whom the test meal exhibited a higher intestinal function regulation (bowel movement) improvement effect, the skin improvement of the test meal was higher. This tendency was made more significant by the test meal (containing collagen peptide and ceramide) in Example 6 than by the test meal in Example 5. By ingesting the test meals in Examples 5 and 6, the elasticity of the skin was increased, and the firmness of the skin was improved.

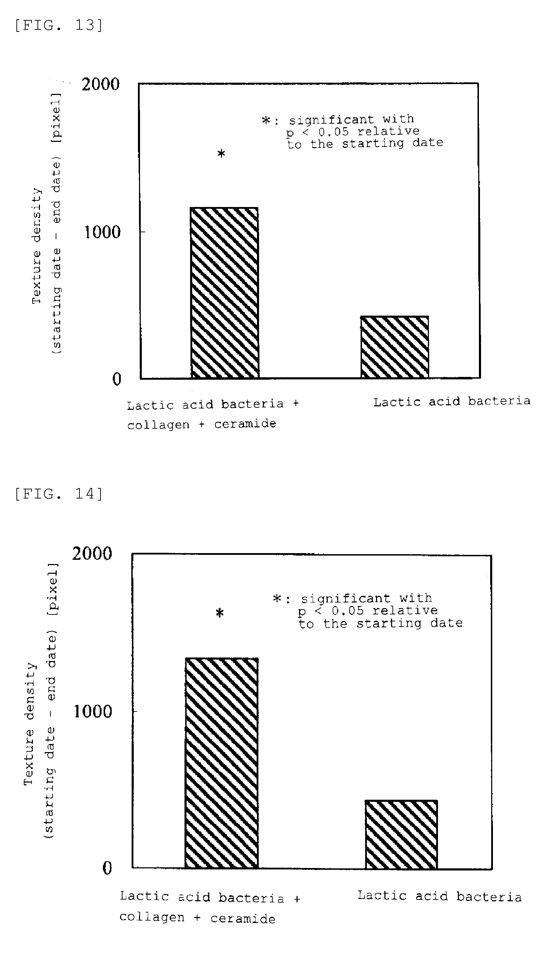

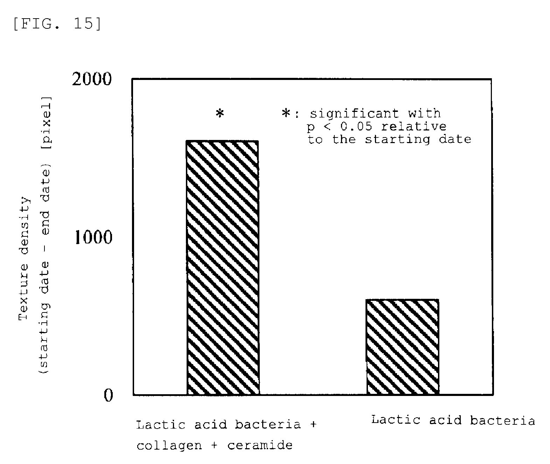

The results of evaluation of the texture density of skin upon ingestion of the test meals in Examples 5 and 6 are shown in FIGS. 13 to 15. FIG. 13 shows the result of evaluation of the texture density of the skin of the subject having intestinal function regulation improved by "1 or more", FIG. 14 shows the result of evaluation of the texture density of the skin of the subject having intestinal function regulation improved by "2 or more", and FIG. 15 shows the result of evaluation of the texture density of the skin of the subject having intestinal function regulation improved by "3 or more". It was demonstrated that for the human for whom the test meal exhibited a higher intestinal function regulation (bowel movement) improvement effect, the skin improvement of the test meal was higher. This tendency was made more significant by the test meal (containing collagen peptide and ceramide) in Example 4 than by the test meal in Example 5. By ingesting the test means in Examples 5 and 6, the texture density of the skin was increased, and the fine texture was improved.

### Industrial Applicability

According to the present invention, there can be provided a composition and fermented milk which are derived from natural products, are highly safe and have a high skin improvement effect and/or treatment effect, comprising lactic acid bacteria excellent in manufacturability. According to the present invention, there can be provided fermented milk having a higher skin improvement effect and treatment effect on humans for whom it exhibited a higher intestinal function regulation improvement effect and/or treatment effect.

### Brief Description of Drawings

FIG. 1 is a graph showing a change in dermatitis development score (expressed in average±standard deviation; **P < 0.01, *P < 0.05, Dunnett' s multiple comparison) during a test period in a distilled water administration group, a *Lactobacillus rhamnosus* ATCC 53103 administration group, a *Streptococcus thermophilus* OLS3059 administration group and a dermatitis non-induction group.
FIG. 2 is a graph showing total IgE level in serum (expressed in average±standard deviation; **P < 0.01, Dunnett's multiple comparison) after 15 days (on Day 15) in a distilled water administration group, a *Lactobacillus rhamnosus* ATCC 53103 administration group, a *Streptococcus thermophilus* OLS3059 administration group and a dermatitis non-induction group.
FIG. 3 is a graph showing a change in dermatitis development score (expressed in average±standard deviation; **P < 0.01, *P < 0.05, Dunnett's multiple comparison) during a test period in a distilled water administration group, a *Lactobacillus bulgaricus* OLL1073R-1 administration group and a *Streptococcus thermophilus* OLS3059 administration group.
FIG. 4 is a graph showing total IgE level in serum (expressed in average±standard deviation; **P < 0.01, Dunnett's multiple comparison) after 15 days (on Day 15) in a distilled water administration group, a *Lactobacillus bulgaricus* OLL1073R-1 administration group and a *Streptococcus thermophilus* OLS3059 administration group.
FIG. 5 is a graph showing a change in dermatitis development score (expressed in average±standard deviation; **P < 0.01, *P < 0.05, Dunnett's multiple comparison) during a test period in a group administered with a fermented product produced with *Lactobacillus bulgaricus* OLL1073R-1 and *Streptococcus thermophilus* OLS3059, a group administered with a fermented product produced with *Lactobacillus delbrueckii* subspecies *bulgaricus* MEP1705601 and *Streptococcus thermophilus* MEP1705601.
FIG. 6 is a graph showing the elasticity of the skin of a subject who ingested, as test meal, fermented milk produced with *Streptococcus thermophilus* OLS3059.

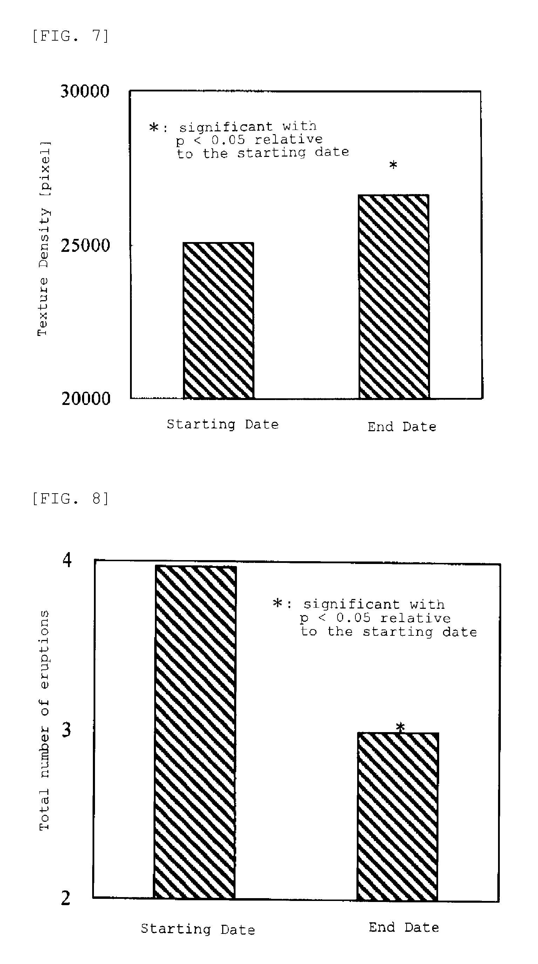

FIG. 7 is a graph showing the texture density of the skin of a subject who ingested, as test meal, fermented milk produced with *Streptococcus thermophilus* OLS3059.
FIG. 8 is a graph showing the observation result of the total number of eruptions in a subject who ingested, as test meal, fermented milk produced with *Streptococcus thermophilus* OLS3059.
FIG. 9 is a graph showing the evaluation result of usefulness by a physician and by a subject who ingested, as test meal, fermented milk produced with *Streptococcus thermophilus* OLS3059.
FIG. 10 is a graph showing the elasticity of the skin of a subject with "intestinal function regulation improved by '1 or more'", who ingested, as test meal, fermented milk produced with *Streptococcus thermophilus* OLS3059.
FIG. 11 is a graph showing the elasticity of the skin of a subject with "intestinal function regulation improved by '2 or more'", who ingested, as test meal, fermented milk produced with *Streptococcus thermophilus* OLS3059.
FIG. 12 is a graph showing the elasticity of the skin of a subject with "intestinal function regulation improved by '3 or more'", who ingested, as test meal, fermented milk produced with *Streptococcus thermophilus* OLS3059.
FIG. 13 is a graph showing the texture density of the skin of a subject with "intestinal function regulation improved by '1 or more'", who ingested, as test meal, fermented milk produced with *Streptococcus thermophilus* OLS3059.
FIG. 14 is a graph showing the texture density of the skin of a subject with "intestinal function regulation improved by '2 or more'", who ingested, as test meal, fermented milk produced with *Streptococcus thermophilus* OLS3059.
FIG. 15 is a graph showing the texture density of the skin of a subject with "intestinal function regulation improved by '3 or more'", who ingested, as test meal, fermented milk produced with *Streptococcus thermophilus* OLS3059.

## Claims

1. Use of *Streptococcus thermophilus* OLS3059 in skin improvement and/or treatment.

2. A composition for skin improvement and/or treatment, which comprises *Streptococcus thermophilus* OLS3059 and/or a culture thereof.

3. The composition for skin improvement and/or treatment according to claim 2, which further comprises *Lactobacillus delbrueckii* subspecies *bulgaricus* OLL1073R-1 and/or a culture thereof.

4. Food and drink comprising the composition of claim 2 or 3.

5. Fermented milk for skin improvement and/or treatment prepared by using *Lactobacillus delbrueckii* subspecies *bulgaricus* and *Streptococcus thermophilus* OLS3059.

6. The fermented milk for skin improvement and/or treatment according to claim 5, which further comprises collagen peptide and/or ceramide.

7. A process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, charging the resulting milk into a container, and fermenting it to form curd followed by cold-storage thereof.

8. A process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, fermenting the resulting milk to form curd, disrupting the curd, and charging it into a container followed by cold-storage thereof.

9. A process for producing the fermented milk for skin improvement and/or treatment of claim 5 or 6, which comprises preparing raw milk, adding *Lactobacillus delbrueckii* subspecies *bulgaricus* and *Streptococcus thermophilus* OLS3059 as starters to the milk, fermenting the milk to form curd, disrupting the curd, cooling it, and charging it into a container followed by cold-storage thereof.
